# EUROPEAN PATENT APPLICATION

(11) **EP 4 603 038 A1**
(43) Date of publication of application: **20.08.2025**
(21) Application number: 23879411.9
(22) Date of filing: 18.07.2023
(51) Int. Cl.: A61B 17/43, A61B 17/435

(54) **OOCYTE COLLECTION NEEDLE AND EMBRYO TRANSFER INSTRUMENT**

(30) Priority: 19.10.2022 JP 2022167508
(71) Applicant: Kitazato Corporation, Fuji-shi, Shizuoka 416-0932 (JP)
(72) Inventor: INOUE, Futoshi, Fuji-shi, Shizuoka 416-0932 (JP)
(74) Representative: Casalonga
(86) International application number: PCT/JP2023/026193
(87) International publication number: WO 2024/084758

(57) **Abstract**

An oocyte retrieval needle 1 includes: a hollow needle 2 that includes a puncture blade surface 23; a hollow grip 3 that includes an indicator protrusion portion 34 that is related to an orientation of the puncture blade surface; and a cylindrical covering portion 33 that covers a portion including the indicator protrusion portion 34. An embryo transfer instrument 10 includes: a flexible tube 6 that includes a tip end curved portion; and a hollow grip 7. The hollow grip 7 includes: an indicator protrusion portion 74 that is related to a curved direction of the flexible tube 6; and a cylindrical covering portion 73 that covers a portion including the indicator protrusion portion 74. The cylindrical covering portions 33 and 73 have high static friction property, transparency, and flexibility. The indicator protrusion portions are visually and tactilely recognizable from an outside of the cylindrical covering portions.

## Description

### Technical Field

The present invention relates to an oocyte retrieval needle for retrieving an oocyte and an embryo transfer instrument used to transfer an ex-vivo fertilized embryo (a fertilized egg) into the uterus of a living body.

### Background Art

An instrument used in reproductive medicine such as an oocyte retrieval needle or an embryo transfer instrument is composed mainly of a tubular body (a needle or a tube) that may include a blade surface or a tip end curved portion at a tip end portion thereof. During and after an operation of inserting the instrument into a living body, it has not been easy to check the direction of the blade surface or the curved direction of the tip end curved portion after the tip end portion of the tubular body has been inserted into the living body.

As the oocyte retrieval needle for retrieving an oocyte, the inventor of the present application has proposed an oocyte retrieval needle in Japanese Utility Model Registration No. 3149897 (Patent Document 1). An oocyte retrieval needle 1 disclosed in Patent Document 1 includes: a hollow needle 2 that includes a hollow main body portion 22 that has a predetermined length and a hollow small diameter tip end portion 21 that extends from the main body portion 22 toward the tip end side and has a length shorter than that of the main body portion 22 and a diameter smaller than that of the main body portion 22; and a hub 34 that is fixed to a basal end portion of the main body portion 22 of the hollow needle 2. Furthermore, at a tip end of the hollow small diameter tip end portion 21, a blade tip that includes a puncture blade surface 23 and a curved side portion that is curved toward the puncture blade surface 23 is provided. The hollow small diameter tip end portion 21 is covered by a low-friction substance, and the hub 34 includes a marker 38 that is related to the orientation of the puncture blade surface 23.

Also, conventionally, an embryo transfer instrument for transferring an ex-vivo fertilized embryo (a fertilized egg) into the uterus of a living body is used. As the embryo transfer instrument, the inventor of the present application has proposed an embryo transfer instrument in JP 2004-129789A (Patent Document 2).

An embryo transfer instrument 1 disclosed in Patent Document 2 includes: a flexible sheath 2 that includes a passage that extends through from a tip end to a rear end thereof and a spherical bulging portion 22 that is provided on an outer surface of the tip end; a flexible stylet 3 that is removably inserted into the flexible sheath and whose tip end slightly protrudes from the tip end surface of the sheath; and a transfer tube body 4 that is insertable into the flexible sheath 2 from which the stylet has been removed, and includes a soft tip end portion 41a that is protrudable from the tip end of the flexible sheath 2 by a predetermined length.

### Citation List

### Patent Documents

Patent Document 1: Japanese Utility Model Registration No. 3149897
Patent Document 2: JP 2004-129789A

### Summary of Invention

### Technical Problem

The inventor of the present application found that, in the oocyte retrieval needle disclosed in Patent Document 1, although the orientation of the blade surface can be easily checked because the marker that is related to the orientation of the puncture blade surface is included in the hub, the marker is formed in the form of a rib, which is tactile but may cause the fingers to slip on the rib surface.

Also, in the embryo transfer instrument disclosed in Cited Document 2, the tip end portion of the flexible sheath 2 has a curved portion. However, after insertion of the curved tip end portion of the flexible sheath 2 into a living body, it has been difficult to confirm the curved direction of the tip end portion of the flexible sheath 2, making it not easy to place the curved tip end portion of the flexible sheath 2 at a favorable orientation.

It is an object of the present invention to provide instruments used in reproductive medicine such as an oocyte retrieval needle and an embryo transfer instrument, specifically, an oocyte retrieval needle and an embryo transfer instrument that each composed mainly of a tubular body (a needle or a tube) with a blade surface or a tip end curved portion at a tip end portion of the tubular body, wherein, during and after an operation of inserting the instrument into a living body, the direction of the blade surface or the curved direction of the tip end curved portion can be easily checked after the tip end portion of the tubular body has been inserted into the living body, and a hollow grip that is fixed to a rear end of the tubular body (the needle or the tube) has favorable gripability during the operation of inserting the instrument into the living body.

### Solution to Problem

The above-described object can be achieved by the following.

An oocyte retrieval needle for retrieving an oocyte from an ovarian follicle comprising: a hollow needle having a blade tip with a puncture blade surface; and a hollow grip is fixed to a rear end portion of the hollow needle, wherein the hollow grip includes: a hollow grip main body; an indicator protrusion portion related to an orientation of the puncture blade surface and extending in a direction of the blade tip of the hollow needle at an axially central portion of the hollow grip main body; and an anti-slip cylindrical cover portion covering the axially central portion of the hollow grip main body including the indicator protrusion portion, the cylindrical cover portion has high static friction property, transparency and flexibility; and the indicator protrusion portion, which is related to the orientation of the puncture blade surface and is covered by the cylindrical cover portion, is visually and tactilely recognizable from an outside of the cylindrical cover portion.

Also, the above-described object can be achieved by the following.

An embryo transfer instrument configured to be used to transfer an embryo into a living body comprising: an outer tube member that includes a flexible tube that includes a passage that extends through from a tip end to a rear end thereof and a hollow grip that is fixed to a rear end portion of the flexible tube; and a stylet member that is removably inserted into the outer tube member and whose tip end is substantially flush with a tip end surface of the flexible tube or slightly protrudes from the tip end surface of the flexible tube, wherein the flexible tube includes a tip end curved portion, the hollow grip includes: a hollow grip main body; an indicator protrusion portion related to a curved direction of the tip end curved portion of the flexible tube and extending in a direction of a tip end of the flexible tube at an axially central portion of the hollow grip main body; and an anti-slip cylindrical cover portion covering the axially central portion of the hollow grip main body including the indicator protrusion portion, the cylindrical cover portion has high static friction property, transparency, and flexibility, and the indicator protrusion portion, which is related to the curved direction of the flexible tube and is covered by the cylindrical cover portion, is visually and tactilely recognizable from an outside of the cylindrical cover portion.

### Brief Description of Drawings

FIG. 1 is a partially omitted front view of an oocyte retrieval needle according to one embodiment of the present invention.
FIG. 2 is a partially omitted external view of the oocyte retrieval needle shown in FIG. 1.
FIG. 3 is a partially omitted enlarged cross-sectional view of the oocyte retrieval needle shown in FIG. 2.
FIG. 4 is an enlarged front view of a hollow grip used in the oocyte retrieval needle shown in FIG. 1.
FIG. 5 is a plan view of the hollow grip shown in FIG. 4.
FIG. 6 is a perspective view of the hollow grip shown in FIG. 4.
FIG. 7 is a cross-sectional view taken along the line A-A shown in FIG. 4.
FIG. 8 is a cross-sectional view taken along the line B-B shown in FIG. 5.
FIG. 9 is a cross-sectional view taken along the line C-C shown in FIG. 4.
FIG. 10 is an enlarged cross-sectional view of a tip end portion of a hollow needle used in the oocyte retrieval needle shown in FIG. 1.
FIG. 11 is an enlarged cross-sectional view of a tip end portion of a hollow needle used in an oocyte retrieval needle according to another embodiment of the present invention.
FIG. 12 is a front view of an embryo transfer instrument according to one embodiment of the present invention.
FIG. 13 is a left side view of the embryo transfer instrument shown in FIG. 12.
FIG. 14 is a front view of a stylet member used in the embryo transfer instrument shown in FIG. 12.
FIG. 15 is an enlarged cross-sectional view of a tip end portion of an outer tube member of the embryo transfer instrument shown in FIG. 12 in a state in which the stylet member is inserted.
FIG. 16 is an enlarged cross-sectional view of a rear end portion of the outer tube member of the embryo transfer instrument shown in FIG. 12 in a state in which the stylet member is inserted, and a rear end portion of the stylet member.
FIG. 17 is a cross-sectional view taken along the line D-D shown in FIG. 16.
FIG. 18 is an enlarged cross-sectional view of a rear end portion of a transfer tube member used in the embryo transfer instrument shown in FIG. 12.
FIG. 19 is a front view of the outer tube member of the embryo transfer instrument shown in FIG. 12 from which the stylet member has been removed and in which the transfer tube member has been inserted.

### Description of Embodiments

The oocyte retrieval needle according to the present invention will be described based on an embodiment shown in the drawings.

An oocyte retrieval needle 1 according to the present invention includes: a hollow needle 2 that includes a blade tip 24 with a puncture blade surface 23 formed at a tip end of the hollow needle 2; and a hollow grip 3 that is fixed to a rear end portion of the hollow needle 2. The hollow grip 3 includes: a hollow grip main body 30; an indicator protrusion portion 34 that is related to an orientation of the puncture blade surface 23, is provided in the hollow grip main body 30, and extends in a direction of the blade tip 24 of the hollow needle 2 at an axially central portion of the hollow grip main body 30; and an anti-slip cylindrical cover portion 33 that covers the axially central portion of the hollow grip main body 30 including the indicator protrusion portion 34. The cylindrical cover portion 33 has high static friction property, transparency, and flexibility. The indicator protrusion portion 34 that is related to the orientation of the puncture blade surface 23 and is covered by the cylindrical cover portion 33 is visually and tactilely recognizable from an outside of the cylindrical cover portion 33.

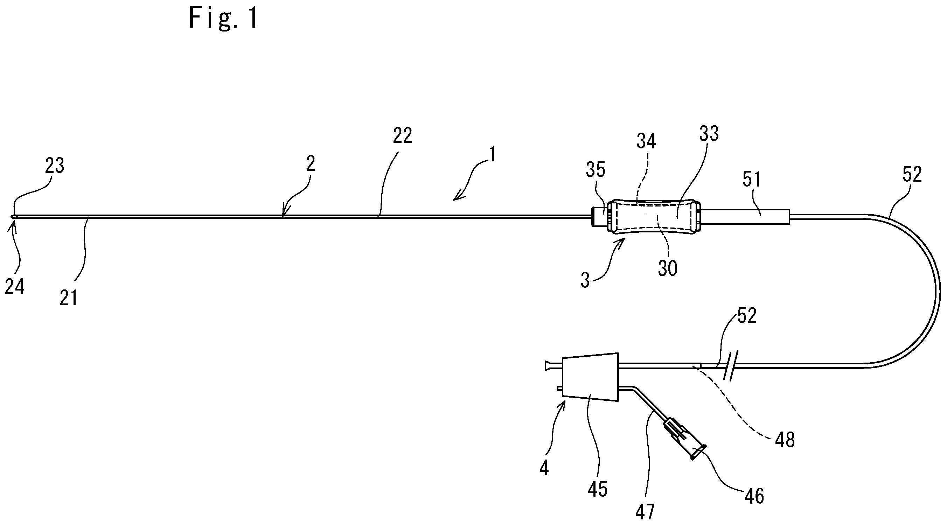

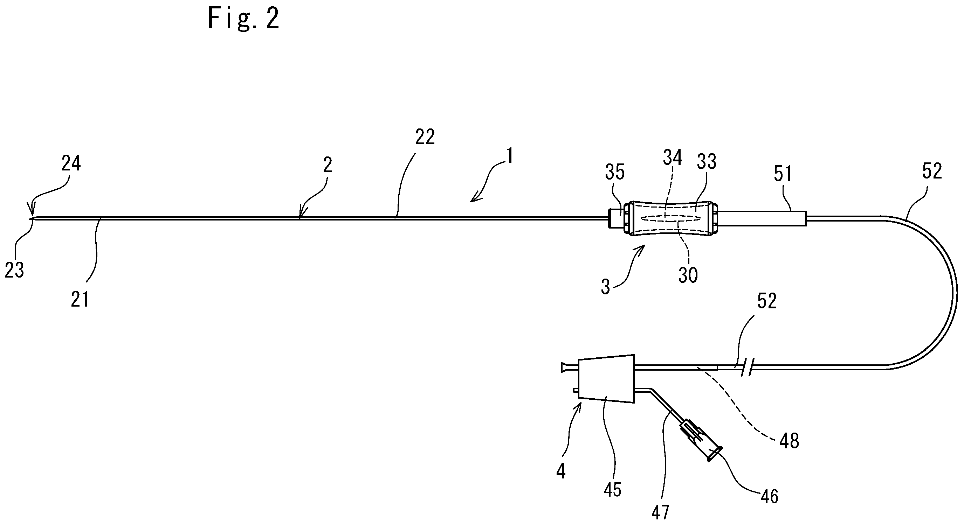

As shown in FIGS. 1 and 2, the oocyte retrieval needle 1 according to the present invention is composed of the hollow needle 2, the hollow grip 3 that is fixed to the rear end portion of the hollow needle 2, and a suction means connection portion 4 that includes a connection tube 52. FIG. 2 is an external view of an assembly of the hollow needle 2 and the hollow grip 3 shown in FIG. 1, rotated by 90 degrees.

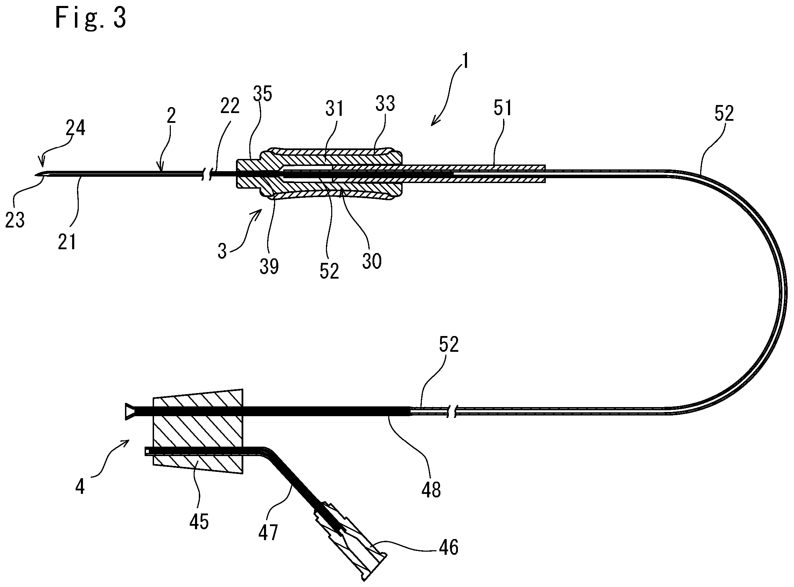

The hollow needle 2 includes the blade tip 24 with the puncture blade surface 23 formed at the tip end of the hollow needle 2 and also includes a lumen that extends from the tip end (the blade tip 24) to the rear end of the hollow needle 2 as shown in FIG. 3. The hollow needle preferably has a length of about 20 to 40 cm, an outer diameter of about 0.50 to 1.50 mm, and an inner diameter of 0.3 to 1.20 mm.

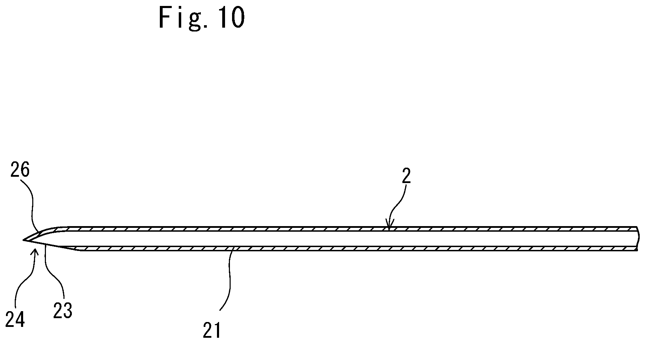

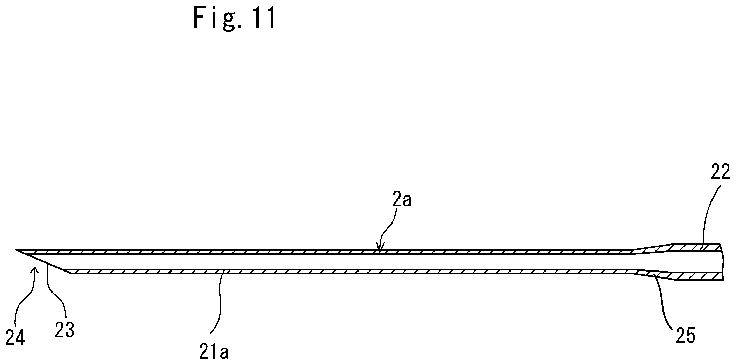

As shown in FIG. 10, the puncture blade surface 23 and the blade tip 24 are formed at the tip end of the hollow needle 2 such that the blade tip 24 has an acute angle with the puncture blade surface 23. The puncture blade surface 23 extends obliquely in a direction of a central axis of the hollow needle 2. The blade tip 24 includes a curved side portion 26 that is curved toward the puncture blade surface 23. As a result of the curved side portion 26 being provided, the blade tip 24 is located slightly closer to the central axis of the hollow needle 2 relative to an extension line of a side surface of a tip end portion 21. As a result of the curved side portion 26 being provided, the puncture blade surface 23 is nearly parallel to the hollow needle 2 and has a sufficient opening area, which makes it easy to perform an oocyte retrieval operation. The blade tip 24 may be an ordinary blade tip that is configured by obliquely cutting the tip end portion of the hollow needle and has no curved side portion as shown in FIG. 11, which will be described later.

The hollow needle may be a hollow needle 2a as shown in FIG. 11. The hollow needle 2a of the present embodiment includes a hollow main body portion 22 that extends in the tip end direction from a basal end thereof by a predetermined length and a small diameter tip end portion 21a that extends toward the tip end side from the main body portion 22. The small diameter tip end portion 21a is configured to have a length shorter than the length of the main body portion 22 and a diameter smaller than the diameter of the main body portion 22. Specifically, as shown in FIG. 11, the small diameter tip end portion 21a is configured to have a smaller diameter than the diameter of the main body portion 22 by being made thinner than the main body portion 22. Also, the small diameter tip end portion 21a has a hollow shape. Accordingly, the hollow needle 2a includes a lumen that extends through from the tip end of the small diameter tip end portion 21a to the basal end of the main body portion 22 as shown in FIG. 3.

The hollow main body portion 22 preferably has a length of about 20 to 30 cm, an outer diameter of about 0.90 to 1.50 mm, and an inner diameter of 0.60 to 1.20 mm. Likewise, the small diameter tip end portion 21a preferably has a length of about 3 to 10 cm, an outer diameter of about 0.50 to 1.00 mm, and an inner diameter of about 0.3 to 0.8 mm.

As a result of the small diameter tip end portion 21a being included in the hollow needle 2a, which is a portion to be penetrated into a subject who undergoes an oocyte retrieval operation, the suffering of the subject during the oocyte retrieval operation can be alleviated, and the penetration resistance during the oocyte retrieval operation can be reduced to a low level. Accordingly, the oocyte retrieval operation can be easily performed. Also, as long as the small diameter tip end portion 21a with a hollow shape is configured to be thinner than the hollow main body portion 22, the penetration resistance can be reduced to an even lower level.

Also, as shown in FIG. 11, a tapered portion 25 is preferably provided between the main body portion 22 and the small diameter tip end portion 21a. As a result of the tapered portion 25 being provided, the penetration resistance of the tapered portion 25 into a subject who undergoes an oocyte retrieval operation can be increased to be sufficiently higher than the penetration resistance of the small diameter tip end portion 21a. Accordingly, it is possible to prevent the tapered portion 25 from being penetrated into the subject. Also, in the present embodiment, the blade tip 24 is an ordinary blade tip that is configured by obliquely cutting the small diameter tip end portion 21a of the hollow needle 2a and has no curved side portion as shown in FIG. 11. In the hollow needle 2a of the present embodiment as well, as with the blade tip shown in FIG. 10, the blade tip 24 may include a curved side portion 26 that is curved toward the puncture blade surface 23.

As the material for forming the hollow needle, a metal tube or a hard synthetic resin tube is used. As the metal of the metal tube, it is preferable to use stainless steel. As the hard synthetic resin of the hard synthetic resin tube, it is preferable to use a fluorine resin such as PTFE or ETFE. Also, the tip end portions 21 and 21a are preferably covered by a low-friction substance on an outer surface thereof. Also, as the low-friction substance, it is preferable to use a silicone oil or a silicone resin. As the silicone oil, it is preferable to use a silicone oil that conforms to silicone oil standards (II: Medical Devices Division of Pharmaceutical Affairs Bureau No. 327, Ministry of Health and Welfare Pharmaceutical Affairs Bureau, Medical Care) or foreign standards equivalent to or higher than the silicone oil standards. As the silicone resin, it is preferable to use a solidified product of a silicone solution composed mainly of dimethylpolysiloxane or the like. As the low-friction substance, a fluorine-based resin such as, for example, PTFE (polytetrafluoroethylene) or ETFE (ethylenetetrafluoroethylene) may be used.

As a result of the tip end portion of the hollow needle, which is a portion to be penetrated into a subject who undergoes an oocyte retrieval operation, being covered by the low-friction substance, the suffering of the subject during the oocyte retrieval operation can be alleviated, and the penetration resistance during the oocyte retrieval operation can be reduced to a low level. Accordingly, the oocyte retrieval operation can be easily performed.

The hollow grip 3 is fixed to the rear end portion of the main body portion 22 of the hollow needle 2. In the present embodiment, a tip end portion of the hollow grip 3 is fixed to the rear end portion of the hollow needle 2 by a fixing portion 39. The rear end portion of the hollow needle 2 extends into the hollow grip 3 beyond the fixing portion 39 that fixes the hollow needle 2 to the hollow grip 3. Furthermore, a tip end portion of the connection tube 52 is fitted to the rear end portion of the hollow needle 2 that extends rearward beyond the fixing portion 39 that fixes the hollow needle 2 to the hollow grip 3. Also, a fixing tube 51 is fitted to the tip end portion of the connection tube 52. A tip end portion of the fixing tube 51 is fixed to the hollow grip 3. Also, the tip end of the connection tube 52 is located near a rear end of the fixing portion 39 of the hollow grip 3. The tip end of the fixing tube 51 is located near an axially central portion of the hollow grip 3, and a rear end of the fixing tube 51 protrudes from a rear end of the hollow grip 3 by a predetermined length.

As shown in FIGS. 3 to 9, the hollow grip 3 includes the hollow grip main body 30 and the cylindrical cover portion 33 that covers the axially central portion of the hollow grip main body 30.

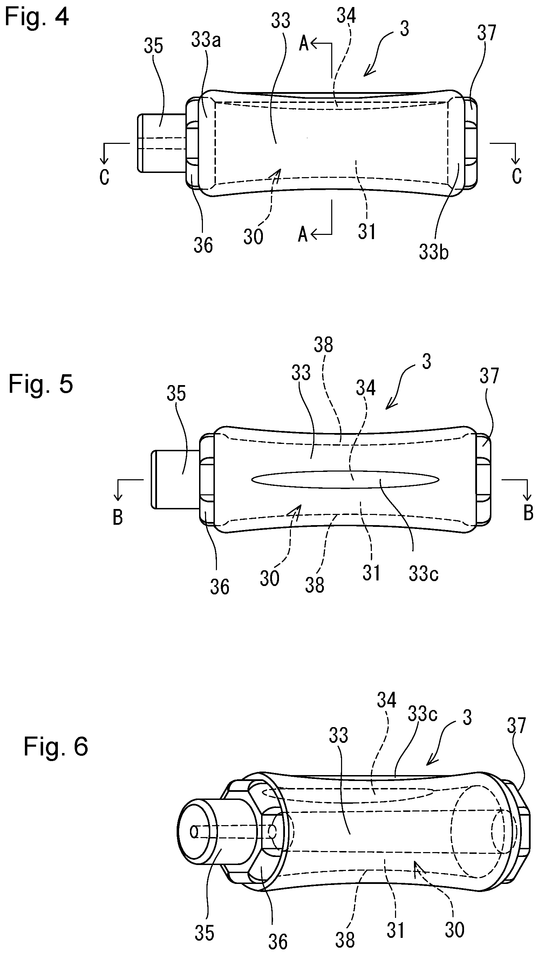

As shown in FIGS. 4 to 9, the hollow grip main body 30 includes: a cylindrical main body portion 31; a small diameter portion 35 that protrudes from a tip end of the cylindrical main body portion 31; a tip end-side annular bulging portion 36 that is provided at the tip end portion of the cylindrical main body portion 31; a rear end-side annular bulging portion 37 that is provided at a rear end portion of the cylindrical main body portion 31; and the indicator protrusion portion 34 that is related to the orientation of the puncture blade surface 23 and extends in the direction of the blade tip 24 of the hollow needle 2 in the axially central portion of the hollow grip main body 30. In the present embodiment, the hollow grip main body 30 has a cylindrical shape.

Also, the cylindrical main body portion 31 (between the tip end-side annular bulging portion 36 and the rear end-side annular bulging portion 37) has a waisted shape whose diameter is reduced from the tip end-side annular bulging portion 36 toward a direction of a smallest diameter portion 38 at an axially central portion of the cylindrical main body portion 31 and is also reduced from the rear end-side annular bulging portion 37 toward the direction of the smallest diameter portion 38 at the axially central portion of the cylindrical main body portion 31, with the smallest diameter portion 38 at the axially central portion of the cylindrical main body portion 31 having the smallest diameter.

Also, the indicator protrusion portion 34 that protrudes outward and extends in the direction of the blade tip 24 of the hollow needle 2 is provided on a side surface of the cylindrical main body portion 31 (between the tip end-side annular bulging portion 36 and the rear end-side annular bulging portion 37) of the hollow grip main body 30. In the present embodiment, the indicator protrusion portion 34 is a rib that extends linearly, with a starting end at a rear end of the tip end-side annular bulging portion 36 and a terminating end at a tip end of the rear end-side annular bulging portion 37. Also, it is preferable that an upper end of the indicator protrusion portion 34 has, from the starting end to the terminating end, the same height with respect to the central axis of the hollow grip main body 30, or in other words, is parallel to the central axis of the hollow grip main body 30.

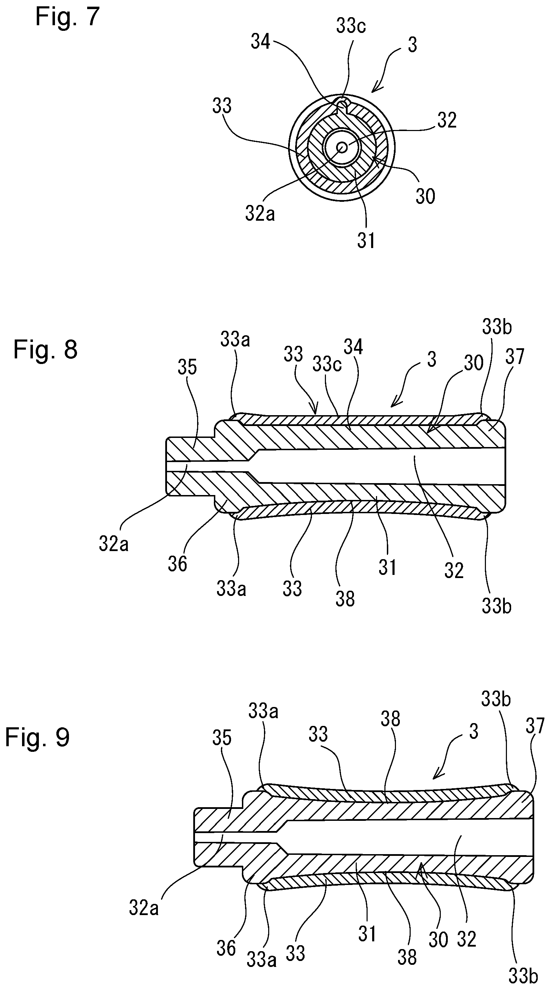

The indicator protrusion portion 34 has a width of preferably 0.5 to 2.5 mm, and more preferably 0.7 to 2.0 mm. Also, an upper surface of the indicator protrusion portion 34 preferably has an edge-free shape as shown in FIG. 7. Specifically, the upper surface of the indicator protrusion portion 34 preferably has a shape with a chamfered or rounded corner, a shape in which an axially orthogonal section of an upper portion of the indicator protrusion portion 34 is semicircular or semi-elliptical, or the like.

Also, as shown in FIGS. 6, 8, and 9, the hollow grip main body 30 includes a lumen 32 that extends through from a tip end to a rear end of the hollow grip main body 30. The lumen 32 includes, at a tip end thereof, a small diameter lumen portion 32a for housing and fixing the rear end portion of the hollow needle 2.

As shown in FIGS. 1 to 9, the indicator protrusion portion 34 that is related to the orientation of the puncture blade surface 23 is formed in an outer surface of the hollow grip 3. The indicator protrusion portion 34 of the present embodiment is formed in the form of a projecting portion that extends linearly from a tip end to a basal end of the hollow grip 3. Also, the indicator protrusion portion 34 extends in parallel to the central axis of the hollow needle 2.

Also, as shown in FIGS. 1 and 2, the puncture blade surface 23 is offset from the position of the indicator protrusion portion 34 by a predetermined angle in a circumferential direction of the hollow needle 2. Specifically, the puncture blade surface 23 is offset from the position of the indicator protrusion portion 34 by approximately 90 degrees in the circumferential direction of the hollow needle 2. As a result of the indicator protrusion portion 34 being provided, the orientation of the puncture blade surface 23 can be easily checked. Furthermore, as long as the puncture blade surface 23 is offset from the position of the indicator protrusion portion 34 of the hollow grip 3 by approximately 90 degrees in the circumferential direction of the hollow needle 2, the orientation of the puncture blade surface 23 can be more easily checked.

The hollow grip main body 30 is preferably formed using a vinyl chloride resin, a polyolefin such as polypropylene or polyethylene, or a synthetic resin such as polycarbonate, polyamide, or polystyrene, in particular, a highly functional thermoplastic resin. Furthermore, the hollow grip main body 30 is preferably formed using a colored opaque material. The color of the colored opaque material may be any color, but is preferably white, yellow, blue, black, or the like.

Also, as shown in FIG. 2, the oocyte retrieval needle 1 according to the present embodiment is configured such that, when the oocyte retrieval needle 1 is held by the right hand of an operator, with the indicator protrusion portion 34 of the hollow grip 3 facing upward, the puncture blade surface 23 faces approximately the left side (palm side), and, when the oocyte retrieval needle 1 is held by the left hand of an operator, the puncture blade surface 23 faces approximately the left side (backhand side). With this configuration, by holding the hollow grip 3 (the indicator protrusion portion 34) to operate the oocyte retrieval needle 1, the orientation of the puncture blade surface can be easily recognized during the oocyte retrieval operation. The orientation of the puncture blade surface can be recognized, which enables ease of operation of the oocyte retrieval needle 1. The indicator protrusion portion 34 of the present embodiment is formed in the form of a projecting portion that extends linearly from the tip end to the basal end of the hollow grip 3. However, the configuration is not limited thereto. The indicator protrusion portion 34 may have any configuration as long as it is possible to check the orientation of the puncture blade surface 23.

Also, the hollow grip main body 3 includes the anti-slip cylindrical cover portion 33 that covers the axially central portion of the hollow grip main body 30 including the indicator protrusion portion 34. The cylindrical cover portion 33 has high static friction property, transparency, and flexibility, and thus the indicator protrusion portion 34 that is related to the orientation of the puncture blade surface 23 and is covered by the cylindrical cover portion 33 is visually and tactilely recognizable from the outside of the cylindrical cover portion 33.

Also, in the oocyte retrieval needle 1, the indicator protrusion portion 34 covered by the cylindrical cover portion 33 forms an indicator raised portion 33c. In other words, as shown in FIGS. 1 to 8, in particular, as shown in FIG. 7, the indicator protrusion portion 34 and a portion of the cylindrical cover portion 33 that covers the indicator protrusion portion 34 are raised as a whole, thereby forming the indicator raised portion 33c. Accordingly, in the indicator protrusion portion 34 and the portion of the cylindrical cover portion 33 that covers the indicator protrusion portion 34, the protrusion portion (raised portion) can be tactilely recognized using a finger. Also, in the present embodiment, the indicator protrusion portion 34 is entirely covered by the cylindrical cover portion 33, and is not exposed at an outer surface thereof.

The cylindrical cover portion 33 is formed using a material that has high static friction property, transparency, and flexibility. As a result of the cylindrical cover portion 33 having high static friction property, it is possible to impart a favorable anti-slip function to the hollow grip 3. Accordingly, the oocyte retrieval needle has favorable operability. Also, as a result of the cylindrical cover portion 33 having transparency, the indicator protrusion portion 34 of the hollow grip 3 can be visually recognized, which enables the operator to easily place his/her thumb on the indicator protrusion portion 34 when holding the oocyte retrieval needle. Also, as a result of the cylindrical cover portion 33 having flexibility, the cylindrical cover portion 33 favorably adheres to the hollow grip main body 30, which prevents the cylindrical cover portion 33 from moving alone and also from being detached from the hollow grip 3. It is more preferable that the cylindrical cover portion 33 has elasticity.

As the material for forming the cylindrical cover portion 33, it is preferable to use a material that is made of an elastic rubber or an elastomer and has transparency. As the transparency, it is sufficient that the indicator protrusion portion 34 can be easily visually recognized from the outside of the cylindrical cover portion 33. Also, the cylindrical cover portion 33 may be configured such that the cylindrical cover portion 33 is fitted in an expanded state to the hollow grip main body 30, and adheres to an outer surface of the hollow grip main body 30 by contraction. With this configuration, the cylindrical cover portion 33 more favorably adheres to the hollow grip main body 30, which reliably prevents the cylindrical cover portion 33 from moving alone and also from being detached from the hollow grip 3.

Also, in the present embodiment, as shown in FIGS. 3 to 9, the hollow grip main body 30 includes the tip end-side annular bulging portion 36 and the rear end-side annular bulging portion 37. A tip end portion 33a of the cylindrical cover portion 33 covers a rear end portion of the tip end-side annular bulging portion 36, and a rear end portion 33b of the cylindrical cover portion 33 covers a tip end portion of the rear end-side annular bulging portion 37 so that two opposite ends of the cylindrical cover portion 33 do not protrude from the hollow grip main body 30. As described above, the two opposite ends of the cylindrical cover portion 33 do not protrude from the hollow grip main body 30, which prevents end portions of the cylindrical cover portion 33 from being separated from the hollow grip main body 30, and reliably prevents the cylindrical cover portion 33 from moving alone relative to the hollow grip main body 30 and also from being detached from the hollow grip 3.

Also, as shown in FIG. 9, the rear end portion of the tip end-side annular bulging portion 36 that is covered by the tip end portion 33a of the cylindrical cover portion 33 has an increased diameter to form an increased diameter portion, and the tip end portion of the rear end-side annular bulging portion 37 that is covered by the rear end portion 33b of the cylindrical cover portion 33 has an increased diameter to form an increased diameter portion. Also, in the present embodiment, the tip end portion 33a of the cylindrical cover portion 33 is configured to be thinner toward the tip end, and the rear end portion 33b of the cylindrical cover portion 33 is configured to be thinner toward the rear end. For this reason, the cylindrical cover portion 33 has a round shape with no corners at the tip end portion 33a and the rear end portion 33b.

Furthermore, in the present embodiment, as described above, the hollow grip main body 30 has a waisted shape whose cross section becomes smaller toward the axially central portion 38 from a tip end portion and a rear end portion of the hollow grip main body 30. Also, the cylindrical cover portion 33 also adheres to an outer surface of the axially central portion 38 of the hollow grip main body 30. Also, in the present embodiment, the cylindrical cover portion 33 is formed using an elastic material and is therefore extendable. As shown in FIG. 7, a portion of the cylindrical cover portion 33 that covers the indicator protrusion portion 34 of the hollow grip main body 30 is extended as compared with the other portion.

As the material for forming the cylindrical cover portion 33, it is possible to use a synthetic rubber such as a urethane rubber, a silicone rubber, or a butadiene rubber, a natural rubber such as a latex rubber, an elastomer such as a polyolefin-based elastomer, a polyamide-based elastomer, or a styrene-based elastomer (for example, a styrene-butadiene-styrene copolymer, a styrene-isoprene-styrene copolymer, or a styrene-ethylene butylene-styrene copolymer), a polyurethane, or the like.

The connection tube 52 is a flexible tube body that has substantially the same outer diameter and substantially the same inner diameter from a tip end to a basal end of the tube body. As the material for forming the connection tube 52 and the fixing tube 51, it is preferable to use a material that has a certain degree of flexibility and a certain degree of firmness. Examples that can be used include polyolefins (for example, polyethylene, polypropylene, and an ethylene-propylene copolymer), polyamides (for example, Nylon 6 and Nylon 66), polyesters (for example, polyethyleneterephthalate), synthetic rubbers such as a urethane rubber, a silicone rubber, and a butadiene rubber, natural rubbers such as a latex rubber, polyolefin-based elastomers, polyamide-based elastomers, and the like.

The connection tube 52 has a length of 100 to 700 mm, and preferably 150 to 250 mm. The connection tube 52 has an outer diameter of 1.0 to 2.0 mm, and preferably 1.46 to 1.79 mm. The connection tube 52 has an inner diameter of 0.7 to 1.5 mm, and preferably 0.86 to 1.19 mm. As long as the connection tube 52 is provided, it is possible to more easily perform the oocyte retrieval operation.

As shown in FIGS. 1 to 3, the suction means connection portion 4 is fixed to a rear end portion of the connection tube 52.

The suction means connection portion 4 includes a stopper 45 that is attached to an opening of a retrieval instrument and a suction means attaching portion 46 that is attached to the stopper 45. Also, the connection tube 52 has one end attached to a basal end of the hollow needle 2 and another end extending through the stopper 45. The other end of the connection tube 52 has an increased diameter to form an increased diameter portion. Also, as shown in FIG. 3, at the rear end portion of the connection tube 52, a tube reinforcement member 48 is provided that extends through the stopper from near the basal end of the connection tube 52 to the tip end side of the tube member by a predetermined length. The reinforcement member 48 is preferably a tubular body that extends within the connection tube 52. Also, the reinforcement member 48 may be a tubular body that extends along an outer surface of the rear end portion of the connection tube 52. As the tubular body, it is preferable to use a hard tubular body or a coil spring. As the material for forming the tubular body, a metal, a synthetic resin, or the like can be used.

Also, the suction means attaching portion 46 includes an attaching portion main body and a shaft portion 47 that extends through the stopper 45 from the main body. The shaft portion 47 is bent. The attaching portion main body has, at a rear end portion thereof, an inner surface shape that is connectable to a connector of a suction device (not shown).

As the material for forming the stopper 45, it is possible to use a synthetic rubber such as a urethane rubber, a silicone rubber, or a butadiene rubber, a natural rubber such as a latex rubber, an elastomer such as a polyolefin-based elastomer, a polyamide-based elastomer, or a styrene-based elastomer (for example, a styrene-butadiene-styrene copolymer, a styrene-isoprene-styrene copolymer, or a styrene-ethylene butylene-styrene copolymer), a polyurethane, or the like. As long as the suction means connection portion 4 is provided, it is possible to more easily perform the oocyte retrieval operation.

The configuration of the suction means connection portion is not limited to the configuration described above. A connection portion (for example, a hub or a connector) that is connectable to a tip portion of a suction means (for example, a syringe) may be provided at a rear end portion of the oocyte retrieval needle, for example, at the rear end of the connection tube 52, at the rear end of the fixing tube 51, or at the rear end of the hollow grip 3.

Next, the embryo transfer instrument according to the present invention will be described based on an embodiment shown in the drawings.

An embryo transfer instrument 10 according to the present invention includes: an outer tube member 11 that includes a flexible tube 6 that includes a passage that extends through from a tip end to a rear end thereof and a hollow grip 7 that is fixed to a rear end portion of the flexible tube 6; and a stylet member 8 that is removably inserted into the outer tube member 11 and whose tip end is substantially flush with a tip end surface of the flexible tube 6 or slightly protrudes from the tip end surface of the flexible tube 6.

The flexible tube 6 includes a tip end curved portion 62. The hollow grip 7 includes: a hollow grip main body 70; an indicator protrusion portion 74 that is related to a curved direction of the tip end curved portion 62 of the flexible tube 6 and extends in a direction of the tip end of the flexible tube 6 at an axially central portion of the hollow grip main body 70; and an anti-slip cylindrical cover portion 73 that covers the axially central portion of the hollow grip main body 70 including the indicator protrusion portion 74. The cylindrical cover portion 73 has high static friction property, transparency, and flexibility, and thus the indicator protrusion portion 74 that is related to the curved direction of the flexible tube 6 and is covered by the cylindrical cover portion 73 can be visually and tactilely recognized from an outside of the cylindrical cover portion 73.

Also, the embryo transfer instrument 10 according to the present embodiment includes an embryo transfer tube member 9 that is insertable into the flexible tube 6 from which the stylet member 8 has been removed, and includes a soft tip end portion that is protrudable from a tip end of the flexible tube 6 by a predetermined length.

Accordingly, the embryo transfer instrument 10 according to the present embodiment includes the outer tube member 11, the stylet member 8, and the embryo transfer tube member 9.

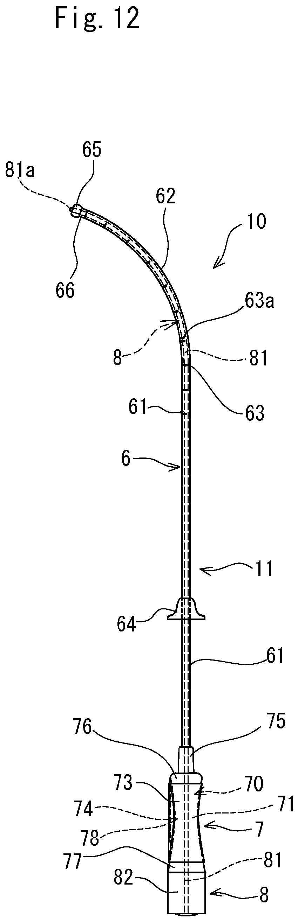

As shown in FIGS. 12, 13, and 15 to 17, the outer tube member 11 includes the flexible tube 6 that includes a passage 67 that extends through from a tip end to a rear end of the flexible tube 6; a spherical bulging portion 65 that is fixed to the tip end of the flexible tube 6 so as not to close a tip end opening of the flexible tube 6; and the hollow grip 7 that is fixed to the rear end of the flexible tube 6.

The flexible tube 6 has a length of 50 to 300 mm, and preferably 100 to 250 mm. The flexible tube 6 has an outer diameter of 1 to 5 mm, and preferably 1.5 to 3.5 mm. The flexible tube 6 has an inner diameter of 0.8 to 4.8 mm, and preferably 1.3 to 3.3 mm. The flexible tube 6 preferably has a certain degree of shape retention ability. As the material for forming the flexible tube 6, a polyester, a polyolefin (for example, polyethylene, polypropylene, or an ethylene-propylene copolymer), a polyamide (for example, Nylon 6 or Nylon 66), a polyester (for example, polyethylene terephthalate), a fluorine resin (for example, PTFE or ETFE), or the like can be used.

Also, the flexible tube 6 includes a linear main body portion 61 and the tip end curved portion 62 that is curved. The tip end curved portion 62 is preferably configured such that a portion that extends from the tip end of the flexible tube 6 by 20 to 100 mm is curved.

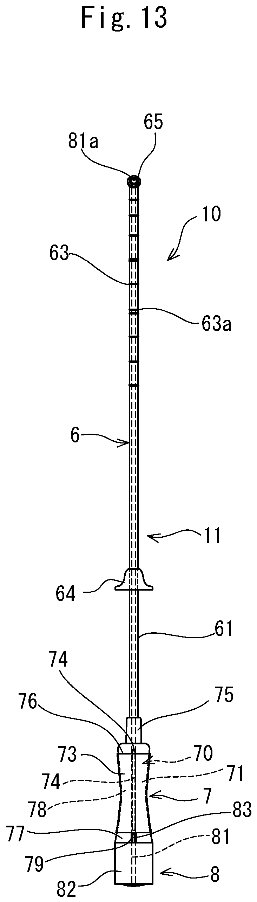

Furthermore, it is preferable that an insertion depth marker 63 is attached to an outer surface of the flexible tube 6 as shown in FIGS. 12 and 13. In the present embodiment, the marker 63 includes a plurality of lines (black lines) that are orthogonal to the central axis of the flexible tube 6. A first marker (line) that is closest to the tip end side of the flexible tube 6 is provided in a tip end side portion of the tip end curved portion 62 (specifically, at a position apart from the tip end of the flexible tube 6 by a predetermined length toward the rear end side of the flexible tube 6), and subsequent markers (lines) provided on the rear end side are spaced apart at a spacing equal to the distance between the first marker and the tip end of the flexible tube 6. A last marker (line) that is closest to the rear end side of the flexible tube 6 is provided at a position apart from the tip end of the main body portion 61 (the tip end of the linear portion) of the flexible tube 6 by a predetermined length toward the rear end side. In particular, in the present embodiment, a double-line marker 63a is provided near the rear end of the tip end curved portion 62. On the tip end side relative to the double-line marker 63a (in the tip end curved portion), a plurality of (specifically, four) markers (lines) are provided. On the rear end side relative to the double-line marker 63a (in the linear main body portion 61) as well, a plurality of (specifically, three) markers (lines) are provided.

Also, in the embryo transfer instrument 10 according to the present embodiment, the spherical bulging portion 65 that is fixed to the tip end of the flexible tube 6 is provided. As shown in FIGS. 12, 13, and 15, the spherical bulging portion 65 is a hollow beadshaped member that includes a passage that extends through the spherical bulging portion 65. The tip end of the flexible tube 6 is inserted into the spherical bulging portion 65, and the spherical bulging portion 65 is fixed to the flexible tube 6. The bulging portion 65 is fixed to the flexible tube 6 using an adhesive, through heat fusion, or the like. The bulging portion 65 preferably has an outer diameter larger than the outer diameter of the tip end portion of the flexible tube 6 by 0.7 to 3 mm, and more preferably by 1 to 2 mm.

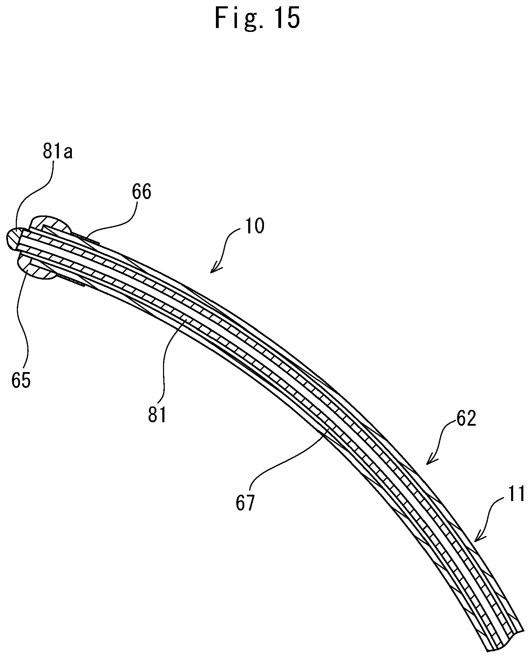

As shown in FIG. 15, the spherical bulging portion 65 is configured to have, at a tip end side thereof, a semi-spherical shape whose diameter decreases toward a tip end of the spherical bulging portion 65. Also, the spherical bulging portion 65 is also configured to have, at a rear end side thereof, a semi-spherical shape whose diameter decreases toward a rear end of the spherical bulging portion 65. As a result of the spherical bulging portion 65 configured as described above being provided, it is possible to prevent damage to the inner wall of the living body during insertion into and removal from the living body. As a result of the spherical bulging portion 65 configured as described above being provided, when the bulging portion abuts against the inner wall of the living body during insertion into the living body, the tip end portion can be favorably escaped. Accordingly, the tip end portion can be easily inserted into a target site in the living body.

Furthermore, in the embryo transfer instrument 10 according to the present embodiment, as shown in FIG. 15, the bulging portion 65 includes, on a basal end side of the inner passage, an increased diameter portion that can house the tip end portion of the flexible tube 6, and also includes, on a tip end side of the inner passage, a small diameter portion that has an inner diameter smaller than the outer diameter of the flexible tube 6. With this configuration, the tip end of the flexible tube 6 is formed by the bulging portion 65 so that the tip end surface of the flexible tube 6 is not exposed. In addition, damage to the inner wall of the living body caused by an outer circumferential edge of the tip end surface of the flexible tube 6 is prevented.

Also, as the material for forming the spherical bulging portion 65, it is preferable to use a hard resin. Furthermore, it is more preferable to use a hard resin that has a small sliding resistance of the outer surface. As the material for forming the bulging portion, any of those listed above as the material for forming the flexible tube 6 can be preferably used.

Furthermore, the flexible tube 6 preferably includes an ultrasound imaging portion 66 that is provided in or behind the bulging portion 65. The ultrasound imaging portion 66 is preferably a ring-shaped member or a coil-shaped member formed using an ultrasound imaging material. As a result of the ultrasound imaging portion 66 configured as described above being provided, the position of the tip end portion of the flexible tube 6, eventually, the position of the bulging portion 65 can be easily checked.

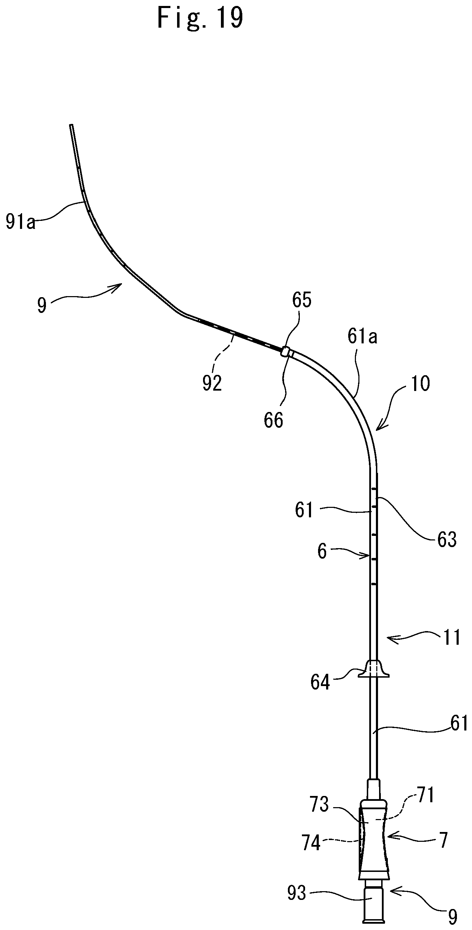

In the embodiment shown in FIGS. 12, 13, and 15, the ultrasound imaging portion 66 is provided on the outer surface of the flexible tube 6 at a position behind the bulging portion 65. The configuration is not limited thereto. As in the embodiment shown in FIG. 19, the ultrasound imaging portion 66 may be provided between the bulging portion 65 and the flexible tube 6. Furthermore, the bulging portion 65 may itself has ultrasound-imaging capability.

Furthermore, the flexible tube 6 preferably includes an insertion depth adjustment member 64 that is movably provided on the outer surface of the flexible tube 6. By providing the adjustment member 64 at a position that corresponds to a desired insertion length in advance, the operation of inserting the flexible tube 6 into the living body can be easily performed.

As shown in FIGS. 12, 13, and 16, the adjustment member 64 of the present embodiment is configured to have a reduced diameter shape in which a tip end side thereof has a diameter reduced toward a tip end of the adjustment member 64. This configuration prevents the subject from suffering from a pain caused by the adjustment member abutting against the inner wall. The adjustment member 64 is a ring-shaped member whose inner diameter is equal to or slightly smaller than the outer diameter of the flexible tube 6. As shown in FIGS. 12, 13, and 16, the adjustment member 64 of the present embodiment includes a flange portion whose diameter is slightly reduced like a tapered shape, a center portion whose diameter is sharply reduced from the flange portion, and a rounded tip end portion. By pressing the adjustment member 64 toward the tip end side of the flexible tube 6 or pulling the adjustment member 64 toward the rear end side of the flexible tube 6 with a certain amount of force, the adjustment member 64 can be slid on the flexible tube 6.

As in the adjustment member of the present embodiment, by configuring the adjustment member 64 to have an edged end surface on the rear end side, it is easy to perform the operation of moving the adjustment member 64 as described above. The adjustment member 64 is preferably formed using an elastic material. As the material for forming the adjustment member 64, it is possible to use, for example, a synthetic rubber such as a urethane rubber, a silicone rubber, or a butadiene rubber, a natural rubber such as a latex rubber, soft vinyl chloride, a polyolefin (polyethylene, polypropylene, an ethylene-propylene copolymer, an ethylene-vinyl acetate copolymer, a mixture of polypropylene with polyethylene or polybutene), a polyester (polyethylene terephthalate or polybutylene terephthalate), a polyamide, an elastomer such as a polyolefin-based elastomer, a polyamide-based elastomer, or a styrene-based elastomer (for example, a styrene-butadiene-styrene copolymer, a styrene-isoprene-styrene copolymer, or a styrene-ethylene butylene-styrene copolymer), a polyurethane, in particular, a thermoplastic polyurethane (thermoplastic polyether polyurethane, thermoplastic polyester polyurethane, in particular, preferably a segmented thermoplastic polyether polyurethane that includes a soft segment and a hard segment, more specifically, the soft segment is preferably composed mainly of polytetramethylene ether glycol, polyethylene glycol, polypropylene glycol, or the like, the hard segment is preferably composed mainly of 1,4-butanediol or the like), or the like.

The hollow grip 7 is fixed to the rear end of the flexible tube 6. The hollow grip 7 is preferably fixed using an adhesive, but may be fixed through fusion, fitting, or the like. The hollow grip 7 is a hollow member that includes a lumen that is in communication with the passage 67 provided in the flexible tube 6.

As shown in FIGS. 12, 13, and 16, the hollow grip 7 includes the hollow grip main body 70 and the cylindrical cover portion 73 that covers the axially central portion of the hollow grip main body 70.

As shown in FIGS. 12, 13, and 16, the hollow grip main body 70 includes a cylindrical main body portion 71, a small diameter portion 75 that protrudes from a tip end of the cylindrical main body portion 71, a tip end-side annular bulging portion 76 that is provided at a tip end portion of the cylindrical main body portion 71, a rear end-side annular bulging portion 77 that is provided at a rear end portion of the cylindrical main body portion 71, and an indicator protrusion portion 74 that is related to the curved direction of the flexible tube 6 and extends in the direction of the tip end of the flexible tube 6 at the axially central portion of the hollow grip main body 70.

Also, the cylindrical main body portion 71 includes a main portion (between the tip end-side annular bulging portion 76 and the rear end-side annular bulging portion 77) that has a waisted shape that includes a narrowest portion 78 at the axially central portion of the hollow grip main body 70, and tapers from the tip end-side annular bulging portion 76 toward the narrowest portion 78 at the axially central portion and also tapers from the rear end-side annular bulging portion 77 toward the narrowest portion 78 at the axially central portion.

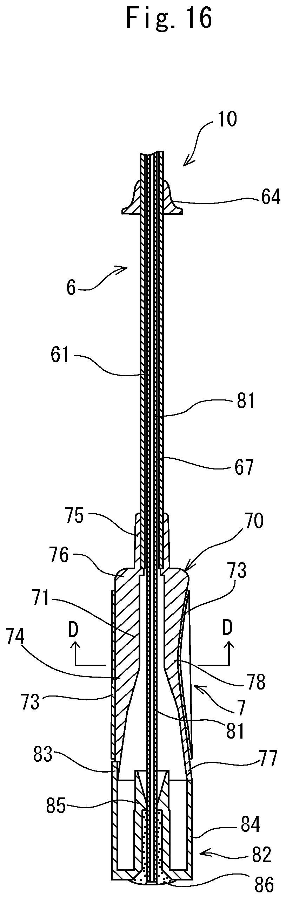

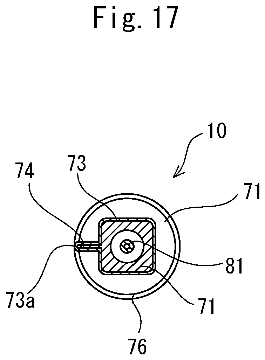

**In** the present embodiment, as shown in FIGS. 16 and 17, a rectangular cylindrical body is used as the hollow grip main body 70. Specifically, the tip end-side annular bulging portion 76 has a cylindrical shape, and a portion extending from a position slightly away from a rear end portion of the tip end-side annular bulging portion 76 to the rear end-side annular bulging portion 77 has an outer shape that is rectangular cylindrical. **In** the inside, the rear end-side annular bulging portion 77 has a rectangular cross section, and a portion on the tip end side from the rear end-side annular bulging portion 77 has a substantially circular cross section.

Also, the indicator protrusion portion 74 that protrudes outward and extends in the direction of the tip end of the flexible tube 6 is provided on a side surface of the main portion (between the tip end-side annular bulging portion 76 and the rear end-side annular bulging portion 77) of the hollow grip main body 70. In the present embodiment, the indicator protrusion portion 74 is a rib that extends linearly, with a starting end at a rear end of the tip end-side annular bulging portion 76 and a terminating end at a tip end of the rear end-side annular bulging portion 77. Also, it is preferable that an upper end of the indicator protrusion portion 74 has, from the starting end to the terminating end, the same height with respect to the central axis of the hollow grip main body 70, or in other words, is parallel to the central axis of the hollow grip main body 70.

The indicator protrusion portion 74 has a width of preferably 0.5 to 2.5 mm, and more preferably 0.7 to 2.0 mm. Also, an upper surface of the indicator protrusion portion 74 preferably has an edge-free shape. Specifically, the upper surface of the indicator protrusion portion 74 preferably has a shape with a chamfered or rounded corner, a shape in which an axially orthogonal section of an upper portion of the indicator protrusion portion 74 is semicircular or semi-elliptical, or the like.

Also, as shown in FIG. 16, the hollow grip main body 70 includes a lumen that extends from a tip end to a rear end of the hollow grip main body 70. A tip end portion (the small diameter portion 75) of the lumen houses the rear end portion of the flexible tube 6, and both of them are fixed.

As shown in FIGS. 12, 13, and 16, the indicator protrusion portion 74 that is related to the curved direction of the flexible tube 6 is provided on an outer surface of hollow grip 7. The indicator protrusion portion 74 of the present embodiment is formed by a projecting portion that protrudes in the curved direction of the tip end curved portion 62 of the flexible tube 6. Specifically, the indicator protrusion portion 74 is a projecting portion that linearly extends from a tip end to a basal end of the hollow grip 7. Also, the indicator protrusion portion 74 extends in parallel to the central axis of the flexible tube 6. As a result of the indicator protrusion portion 74 being provided, the curved direction of the tip end curved portion 62 of the flexible tube 6 can be easily checked.

The hollow grip main body 70 is preferably formed using a vinyl chloride resin, a polyolefin such as polypropylene or polyethylene, or a synthetic resin such as polycarbonate, polyamide, or polystyrene, in particular, a highly functional thermoplastic resin. Furthermore, the hollow grip main body 70 is preferably formed using a colored opaque material. The color of the colored opaque material may be any color, but is preferably white, yellow, blue, black, or the like.

Also, as shown in FIG. 12, the embryo transfer instrument 10 according to the present embodiment is configured such that, when the embryo transfer instrument 10 is placed such that the tip end curved portion 62 of the flexible tube 6 is flat, the indicator protrusion portion 74 faces in the curved direction of the tip end curved portion 62 of the flexible tube 6. For this reason, as shown in FIG. 13, when the operator holds the hollow grip 7 by pressing the indicator protrusion portion 74 with his/her thumb, the curved direction of the tip end curved portion 62 of the flexible tube 6 and the tip end of the flexible tube 6 face in a direction of the thumb of the operator. With this configuration, the curved direction of the tip end curved portion 62 of the flexible tube 6 and the orientation of the tip end of the flexible tube 6 can be easily recognized during embryo transfer procedure, which enables ease of operation of the embryo transfer instrument 10. The indicator protrusion portion 74 of the present embodiment is configured in the form of a projecting portion that extends linearly from the tip end to the basal end of the hollow grip 7. However, the configuration is not limited thereto. The indicator protrusion portion 74 may have any configuration as long as it is possible to check the curved direction of the flexible tube 6.

Also, the hollow grip 7 includes the anti-slip cylindrical cover portion 73 that covers the axially central portion of the hollow grip main body 70 including the indicator protrusion portion 74. The cylindrical cover portion 73 has high static friction property, transparency, and flexibility, thus the indicator protrusion portion 74 that is related to the curved direction of the flexible tube 6 and is covered by the cylindrical cover portion 73 can be visually and tactilely recognized from the outside of the cylindrical cover portion 73.

Also, in the embryo transfer instrument 10, the indicator protrusion portion 74 covered by the cylindrical cover portion 73 forms an indicator raised portion 73a. In other words, as shown in FIGS. 12, 13, 16, and 17, the indicator protrusion portion 74 and a portion of the cylindrical cover portion 73 that covers the indicator protrusion portion 74 are raised as a whole, thereby forming the indicator raised portion 73a. Accordingly, in the indicator protrusion portion 74 and the portion of the cylindrical cover portion 73 that covers the indicator protrusion portion 74, the protrusion portion (raised portion) can be tactilely recognized using a finger. Also, in the present embodiment, the indicator protrusion portion 74 is entirely covered by the cylindrical cover portion 73, and is not exposed at an outer surface thereof.

The cylindrical cover portion 73 is formed using a material that has high static friction property, transparency, and flexibility. As a result of the cylindrical cover portion 73 having high static friction property, it is possible to impart a favorable anti-slip function to the hollow grip 7. Accordingly, the embryo transfer instrument has a favorable operability. Also, as a result of the cylindrical cover portion 73 having transparency, the indicator protrusion portion 74 of the hollow grip 7 can be visually recognized, which enables the operator to easily place his/her thumb on the indicator protrusion portion 74 when holding the embryo transfer instrument. Also, as a result of the cylindrical cover portion 73 having flexibility, the cylindrical cover portion 73 favorably adheres to the hollow grip main body 70, which prevents the cylindrical cover portion 73 from moving alone and also from being detached from the hollow grip 7. It is more preferable that the cylindrical cover portion 73 has elasticity.

As the material for forming the cylindrical cover portion 73, it is preferable to use a material that is made of an elastic rubber or an elastomer and has transparency. As the transparency, it is sufficient that the indicator protrusion portion 74 can be easily visually recognized from the outside of the cylindrical cover portion 73. Also, the cylindrical cover portion 73 may be configured such that the cylindrical cover portion 73 is fitted in an expanded state to the hollow grip main body 70, and adheres to an outer surface of the hollow grip main body 70 by contraction. With this configuration, the cylindrical cover portion 73 more favorably adheres to the hollow grip main body 70, which reliably prevents the cylindrical cover portion 73 from moving alone and also from being detached from the hollow grip 7.

Also, in the present embodiment, as shown in FIGS. 12, 13, and 16, the hollow grip main body 70 includes the tip end-side annular bulging portion 76 and the rear end-side annular bulging portion 77. Also, as shown in FIGS. 12, 13, and 16, in the hollow grip 7, a stylet hub 82 of the stylet member 8, which will be described later, is attached to a rear end portion of the hollow grip main body 70. The outer tube member 11 and the stylet member 8 include rotation restriction engaging portions 83 and 79 that restrict relative rotation between the outer tube member 11 and the stylet member 8. Specifically, a projection housing recess 79 that houses a projection 83 provided on the stylet hub 82 is formed at the rear end portion of the hollow grip main body 70. As a result of the projection 83 and the projection housing recess 79 being engaged with each other, the relative rotation between the projection 83 and the projection housing recess 79 is restricted.

A tip end portion of the cylindrical cover portion 73 covers a rear end portion of the tip end-side annular bulging portion 76, and a rear end portion of the cylindrical cover portion 73 covers a tip end portion of the rear end-side annular bulging portion 77 so that two opposite ends of the cylindrical cover portion 73 do not protrude from the hollow grip main body 70. As described above, the two opposite ends of the cylindrical cover portion 73 do not protrude from the hollow grip main body 70, which prevents end portions of the cylindrical cover portion 73 from being separated from the hollow grip main body 70, and reliably prevents the cylindrical cover portion 73 from moving alone relative to the hollow grip main body 70 and also from being detached from the hollow grip 7.

Also, the embryo transfer instrument 10 according to the present embodiment may also be configured such that, as with the hollow grip 3 of the oocyte retrieval needle 1 described above, the rear end portion of the tip end-side annular bulging portion that is covered by the tip end portion of the cylindrical cover portion has an increased diameter to form an increased diameter portion, and the tip end portion of the rear end-side annular bulging portion that is covered by the rear end portion of the cylindrical cover portion has an increased diameter to form an increased diameter portion as shown in FIG. 9. Also, the tip end portion of the cylindrical cover portion may be configured to be thinner toward the tip end, and the rear end portion of the cylindrical cover portion may be configured to be thinner toward the rear end. In this case, the cylindrical cover portion has a round shape with no corners at the tip end portion and the rear end portion.

Furthermore, in the present embodiment, as described above, the hollow grip main body 70 has a waisted shape whose cross section becomes smaller toward the narrowest portion (axially central portion) 78 from the tip end portion and the rear end portion of the hollow grip main body 70. Also, the cylindrical cover portion 73 also adheres to an outer surface of the narrowest portion (axially central portion) 78 of the hollow grip main body 70. Also, in the present embodiment, the cylindrical cover portion 73 is formed using an elastic material and is therefore extendable. As shown in FIG. 7, a portion of the cylindrical cover portion 73 that covers the indicator protrusion portion 74 of the hollow grip main body 70 is extended as compared with the other portion.

As the material for forming the cylindrical cover portion 73, it is possible to use a synthetic rubber such as a urethane rubber, a silicone rubber, or a butadiene rubber, a natural rubber such as a latex rubber, an elastomer such as a polyolefin-based elastomer, a polyamide-based elastomer, or a styrene-based elastomer (for example, a styrene-butadiene-styrene copolymer, a styrene-isoprene-styrene copolymer, or a styrene-ethylene butylene-styrene copolymer), a polyurethane, or the like.

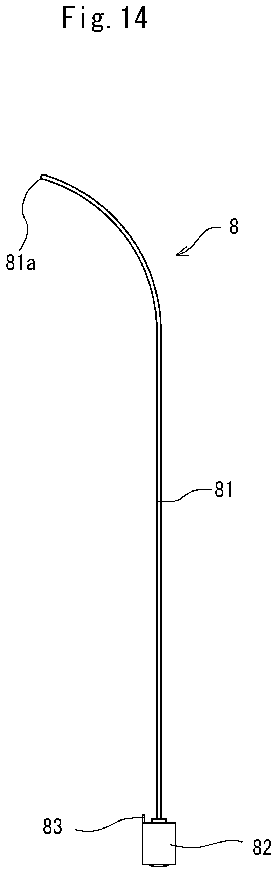

As shown in FIGS. 14 and 16, the stylet member 8 includes a shaft-shaped stylet main body 81 and the stylet hub 82 that is fixed to a rear end of the stylet member 8. As a result of the stylet member 8 being provided, it is possible to prevent a secretion and the like from the living body from entering the flexible tube 6 during insertion of the embryo transfer instrument 10 into the living body. Also, in the embryo transfer instrument 10 according to the present embodiment, the stylet main body 81 is configured such that, in a state in which the stylet main body 81 is inserted into the flexible tube 6 and the stylet hub 82 is engaged with the hollow grip 7, a tip end of the stylet main body 81 slightly protrudes from the tip end of the flexible tube 6. The protruding length of the stylet main body 81 from the flexible tube 6 is preferably 5 mm or less, and more preferably 2 mm or less. The tip end of the stylet main body 81 may be located at substantially the same position as the tip end surface of the flexible tube 6.

As shown in FIGS. 13 to 15, the stylet member 81 is configured such that a tip end 81a of the stylet member 81 has a semi-spherical shape whose diameter is reduced toward a tip end of the stylet member 81. For this reason, it is possible to prevent damage to the inner wall of the living body during insertion into and removal from the living body. Also, in the present embodiment, the stylet main body 81 is formed using a tube-shaped member. The stylet main body may be formed using a solid member. As the material for forming the stylet main body, any of those listed above as the material for forming the flexible tube 6 and those listed above as the material for forming the adjustment member 64 can be used. It is preferable that the stylet main body 81 has an inner diameter smaller than the inner diameter of the flexible tube 6 by 0.1 to 0.5 mm, and preferably 0.1 to 0.2 mm.

As shown in FIG. 15, the stylet hub 82 is fixed to a rear end of the stylet main body 81 using an adhesive. The stylet hub 82 includes a passage that houses and fixes a rear end portion of the stylet main body 81, and also includes an inner cylinder portion 85 that can be inserted into a rear end portion 77 of the hollow grip 7 and an outer cylinder portion 84 that encloses the inner cylinder portion 85. The outer cylinder portion 84 includes a hub engaging projection 83 that protrudes toward the tip end side. The hub engaging projection 83 engages with the projection housing recess 79 of the hollow grip 7. As a result of the hub engaging projection 83 and the projection housing recess 79 being engaged with each other, the rotation of the stylet member 8 relative to the outer tube member 11 is restricted.

The embryo transfer instrument 10 according to the present embodiment includes an embryo transfer tube member 9 that is insertable, from the outer tube member 11, into the flexible tube 6 from which the stylet member 8 has been removed, and includes a soft tip end portion that is protrudable from the tip end of the flexible tube 6 by a predetermined length.

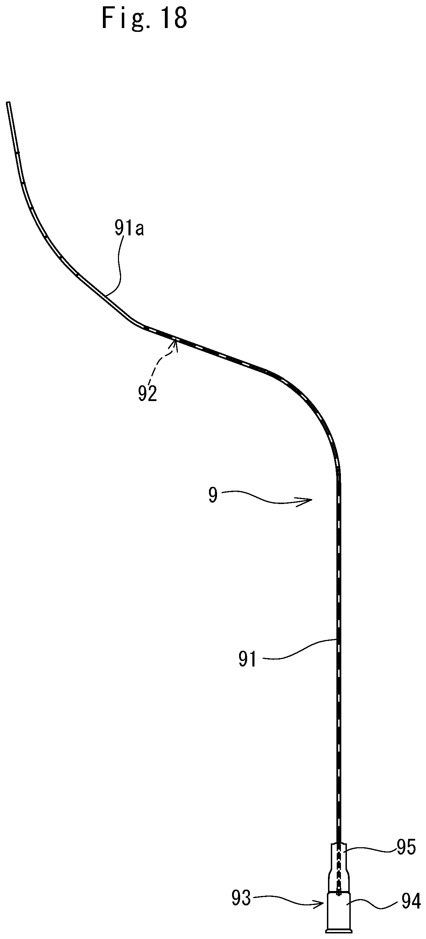

As shown in FIGS. 18 and 19, the embryo transfer tube member 9 that is inserted into the flexible tube 6 after the stylet member has been removed includes a soft tip end portion 91a that is protrudable from the tip end of the flexible tube 6 by a predetermined length. The tube member 9 of the present embodiment includes a soft tube 91, a rigidity imparting tube member 92 that is inserted into the soft tube 91 and whose tip end is located at a position away from a tip end of the soft tube 91 toward the rear end side by a predetermined length, and a tube hub 93 that is fixed to a rear end of the soft tube 91.

The embryo transfer tube member 9 is a tube for transferring embryos and includes a passage that extends from a tip end to a rear end of the embryo transfer tube member 9. In a portion where the rigidity imparting tube member 92 is present, the passage is formed within the tube member 92, and in a tip end portion of the embryo transfer tube member 9 where the tube member 92 is not present, the passage is formed within the soft tube 91.

The embryo transfer tube member 9 has a length of about 70 to 800 mm, and in particular, preferably about 200 to 600 mm. The embryo transfer tube member 9 has an outer diameter of about 0.5 to 3 mm, and in particular, preferably about 1 to 2 mm. Also, the soft tube 91 has an inner diameter of about 0.3 to 0.7 mm, and in particular, preferably about 0.4 to 0.6 mm.

As the material for forming the soft tube 91, it is preferable to use a material that has a certain degree of flexibility. It is possible to use, for example, a synthetic rubber such as a urethane rubber, a silicone rubber, or a butadiene rubber, a natural rubber such as a latex rubber, soft vinyl chloride, a polyolefin (polyethylene, polypropylene, an ethylene-propylene copolymer, an ethylene-vinyl acetate copolymer, a mixture of polypropylene with polyethylene or polybutene), a polyester (polyethylene terephthalate or polybutylene terephthalate), a polyamide, an elastomer such as a polyolefin-based elastomer, a polyamide-based elastomer, or a styrene-based elastomer (for example, a styrene-butadiene-styrene copolymer, a styrene-isoprene-styrene copolymer, or a styrene-ethylene butylene-styrene copolymer), a polyurethane, in particular, a thermoplastic polyurethane (thermoplastic polyether polyurethane, thermoplastic polyester polyurethane, in particular, preferably a segmented thermoplastic polyether polyurethane that includes a soft segment and a hard segment, more specifically, the soft segment is preferably composed mainly of polytetramethylene ether glycol, polyethylene glycol, polypropylene glycol, or the like, the hard segment is preferably composed mainly of 1,4-butanediol or the like), or the like. It is preferable to use a rubber such as a silicone rubber, or an elastomer. In particular, a silicone rubber is preferable. As the silicone rubber, a silicone rubber that has a 200% modulus of about 30 to 90 kg/cm², and preferably 40 to 60 kg/cm² is used.

The rigidity imparting tube member 92 is preferably provided such that a tip end of the rigidity imparting tube member 92 is located at a position away from the tip end of the soft tube 91 toward a basal end side of the tube member 9 by a length of 5 to 150 mm. In particular, the length is preferably 10 to 100 mm. Accordingly, the tip end portion of the embryo transfer tube member 9 is formed only by the soft tube 91. This configuration is preferable because it causes less damage to the inner wall of the living body during insertion of the embryo transfer instrument 10 into the living body. In addition, due to the rigidity imparting tube member 92 being provided, a pushing force applied to the basal end side of the tube member 9 is favorably transmitted, and thus the operation of inserting the embryo transfer tube member 9 to a target site can be easily performed. The length of the rigidity imparting tube member 92 is preferably set such that, when the embryo transfer tube member 9 is inserted into the flexible tube 6, a tip end portion of the rigidity imparting tube member 92 can protrude from the tip end portion of the flexible tube 6. Also, the rigidity imparting tube member 92 preferably has an outer diameter that is substantially equal to the inner diameter of the soft tube 91 or smaller than the inner diameter of the soft tube 91 by about 0.1 to 0.5 mm.

As the material for forming the rigidity imparting tube member 92, a polyester, a polyolefin (for example, polyethylene, polypropylene, or an ethylene-propylene copolymer), a polyamide (for example, Nylon 6 or Nylon 66), a polyester (for example, polyethylene terephthalate), a fluorine resin (for example, PTFE or ETFE), or the like can be used.

Also, the embryo transfer tube member 9 includes the tube hub 93. The tube hub 93 includes a hub main body 94 and an anti-kink member 95. The soft tube 91 is fixed to the hub main body 94 by a crimping member (not shown) inserted into the rear end. A rear end of the rigidity imparting tube member 92 is inserted into the crimping member. The rigidity imparting tube member 92 is fixed to the hub in the tube hub that includes the crimping member, or is fixed to the soft tube by an adhesive or the like in the soft tube. The tube hub 93 is configured such that an injection instrument such as a syringe can be attached to a rear end of the tube hub 93. Specifically, an inner surface of the hub main body 94 is a luer taper portion to which a tip end portion of a syringe can be liquid-tightly attached.

An operational function of the embryo transfer instrument 10 according to the present invention will be described.

An oocyte is retrieved transvaginally or laparoscopically using the oocyte retrieval needle, and the retrieved oocyte is placed in a culture vessel that contains a culture solution, and then cultured. A retrieved sperm is, for example, concentrated by precipitation using a centrifuge, resuspended, and added to a culture solution. The resultant is added to the oocyte culture solution that has been cultured for 4 to 6 hours to cause insemination. Then, male and female pronuclei are observed to confirm fertilization. If male and female pronuclei (two pronuclei) are observed, it is determined as a normally fertilized embryo (fertilized egg), and the culture is continued. If three or more pronuclei are observed, it is highly likely that it indicates polyspermic fertilization. Accordingly, the pronuclei are removed, and degenerated eggs are also removed. A fertilized embryo transfer is possible. However, normally, a fertilized embryo is cultured, and an embryo that has split into two, four, or eight embryos is fertilized. In general, a 4-cell stage embryo that has split into four embryos about 44 hours after insemination is used in embryo transfer.

A syringe (not shown) filled with the same culture solution as that used to culture the fertilized egg is attached to the hub main body 94 of the embryo transfer tube member 9 of the present invention, and the lumen of the tube member 9 is filled with the culture solution. Then, the tip end of the tube member 9 is inserted into the culture vessel, the tip end of the instrument is caused to abut against the bottom surface of the culture vessel near the embryo, and thereafter, the embryo is drawn into the lumen of the tube member 9 by being suctioned using the syringe. There is an opening in the tip end surface of the tube member 9, and thus the embryo suction operation can be easily performed.

Then, the outer tube member 11 to which the stylet member 8 has been attached is inserted through the vaginal opening and passed through the cervix. Next, the embryo transfer tube member 9 that contains the embryo in the flexible tube 6 of the outer tube member 11 is inserted and pushed such that the tip end portion reaches an opening of the cervix, and thereafter, the syringe is pushed to transfer the embryo into the uterus. Then, the tube member 9 and the outer tube member 11 are removed. In this way, the embryo transfer ends.

The oocyte retrieval needle according to the present invention includes: a hollow needle that includes a blade tip with a puncture blade surface; and a hollow grip that is fixed to a rear end portion of the hollow needle, wherein the hollow grip includes: a hollow grip main body; an indicator protrusion portion that is related to an orientation of the puncture blade surface and extends in a direction of the blade tip of the hollow needle at an axially central portion of the hollow grip main body; and an anti-slip cylindrical cover portion that covers the axially central portion of the hollow grip main body including the indicator protrusion portion, the cylindrical cover portion has high static friction property, transparency, and flexibility, and the indicator protrusion portion that is related to the orientation of the puncture blade surface and is covered by the cylindrical cover portion is visually and tactilely recognizable from an outside of the cylindrical cover portion.

The oocyte retrieval needle has favorable operability because, due to the axially central portion of the hollow grip main body including the indicator protrusion portion being covered by the anti-slip cylindrical cover portion, the fingers do not slip on the indicator protrusion portion when holding the hollow grip. In addition, the cylindrical cover portion has high static friction property, transparency, and flexibility, and the indicator protrusion portion that is related to the orientation of the puncture blade surface and is covered by the cylindrical cover portion can be visually and tactilely recognized from the outside of the cylindrical cover portion, and thus the orientation of the puncture blade surface can be easily checked.

Also, the embryo transfer instrument according to the present invention includes: an outer tube member that includes a flexible tube that includes a passage that extends through from a tip end to a rear end thereof and a hollow grip that is fixed to a rear end portion of the flexible tube; and a stylet member that is removably inserted into the outer tube member and whose tip end is substantially flush with a tip end surface of the flexible tube or slightly protrudes from the tip end surface of the flexible tube, wherein the flexible tube includes a tip end curved portion, the hollow grip includes: a hollow grip main body; an indicator protrusion portion that is related to a curved direction of the flexible tube and extends in a direction of the tip end of the flexible tube at an axially central portion of the hollow grip main body; and an anti-slip cylindrical cover portion that covers the axially central portion of the hollow grip main body including the indicator protrusion portion, the cylindrical cover portion has high static friction property, transparency, and flexibility, and the indicator protrusion portion that is related to the curved direction of the flexible tube and is covered by the cylindrical cover portion is visually and tactilely recognizable from an outside of the cylindrical cover portion.

The embryo transfer instrument has favorable operability because, due to the axially central portion of the hollow grip main body including the indicator protrusion portion being covered by the anti-slip cylindrical cover portion, the fingers do not slip on the indicator protrusion portion when holding the hollow grip. In addition, the cylindrical cover portion has high static friction property, transparency, and flexibility, and the indicator protrusion portion that is related to the curved direction of the flexible tube and is covered by the cylindrical cover portion can be visually and tactilely recognized from the outside of the cylindrical cover portion, and thus the curved direction of the flexible tube can be easily checked.

### Industrial Applicability

The oocyte retrieval needle according to the present invention is as follows.
(1) An oocyte retrieval needle for retrieving an oocyte from an ovarian follicle comprising: a hollow needle having a blade tip with a puncture blade surface; and a hollow grip is fixed to a rear end portion of the hollow needle, wherein the hollow grip includes: a hollow grip main body; an indicator protrusion portion related to an orientation of the puncture blade surface and extending in a direction of the blade tip of the hollow needle at an axially central portion of the hollow grip main body; and an anti-slip cylindrical cover portion covering the axially central portion of the hollow grip main body including the indicator protrusion portion, the cylindrical cover portion has high static friction property, transparency and flexibility; and the indicator protrusion portion, which is related to the orientation of the puncture blade surface and is covered by the cylindrical cover portion, is visually and tactilely recognizable from an outside of the cylindrical cover portion.

The oocyte retrieval needle has favorable operability because, due to the axially central portion of the hollow grip main body including the indicator protrusion portion being covered by the anti-slip cylindrical cover portion, the fingers do not slip on the indicator protrusion portion when holding the hollow grip. In addition, the cylindrical cover portion has high static friction property, transparency, and flexibility, and the indicator protrusion portion that is related to the orientation of the puncture blade surface and is covered by the cylindrical cover portion can be visually and tactilely recognized from the outside of the cylindrical cover portion, and thus the orientation of the puncture blade surface can be easily checked.

Also, the embodiment described above may be configured as follows
(2) The oocyte retrieval needle of the above (1), wherein the puncture blade surface is offset from a position of the indicator protrusion portion of the hollow grip by approximately 90 degrees in a circumferential direction of the hollow needle so that, when the oocyte retrieval needle is held with the hollow grip being positioned on the right side, the blade tip being positioned on the left side, and the indicator protrusion portion facing upward, the puncture blade surface substantially faces a front side.
(3) The oocyte retrieval needle of the above (1) or (2), wherein the indicator protrusion portion covered by the cylindrical covering portion forms an indicator raised portion.
(4) The oocyte retrieval needle according to any one of the above (1) to (3),
   wherein the cylindrical covering portion is formed using an elastic rubber or an elastomer.
(5) The oocyte retrieval needle according to any one of the above (1) to (4), wherein the cylindrical covering portion is formed using a transparent material or a translucent material, and the hollow grip main body is formed using a colored opaque material.
(6) The oocyte retrieval needle according to any one of the above (1) to (5), wherein the cylindrical covering portion is fitted in an expanded state to the hollow grip main body, and adheres to an outer surface of the hollow grip main body by contraction.
(7) The oocyte retrieval needle according to any one of the above (1) to (6), wherein the hollow grip main body includes a tip end-side annular bulging portion and a rear end-side annular bulging portion, and a tip end portion of the cylindrical covering portion covers a rear end portion of the tip end-side annular bulging portion and a rear end portion of the cylindrical covering portion covers a tip end portion of the rear end-side annular bulging portion so that two opposite ends of the cylindrical covering portion do not protrude from the hollow grip main body.
(8) The oocyte retrieval needle according to the above (7), wherein the rear end portion of the tip end-side annular bulging portion that is covered by the tip end portion of the cylindrical covering portion has an increased diameter to form an increased diameter portion, and the tip end portion of the rear end-side annular bulging portion that is covered by the rear end portion of the cylindrical covering portion has an increased diameter to form an increased diameter portion.
(9) The oocyte retrieval needle according to the above (7) or (8), wherein the tip end portion of the cylindrical covering portion has a thickness that decreases toward a tip end thereof, and the rear end portion of the cylindrical covering portion has a thickness that decreases toward a rear end thereof.
(10) The oocyte retrieval needle according to any one of the above (1) to (9), wherein the hollow grip main body has a cross section that decreases toward the axially center portion from a tip end portion and a rear end portion of the hollow grip main body, and the cylindrical covering portion adheres to an outer surface of the axially center portion of the hollow grip main body.

The embryo transfer instrument according to the present invention is as follows.
(11) An embryo transfer instrument configured to be used to transfer an embryo into a living body comprising: an outer tube member that includes a flexible tube that includes a passage that extends through from a tip end to a rear end thereof and a hollow grip that is fixed to a rear end portion of the flexible tube; and a stylet member that is removably inserted into the outer tube member and whose tip end is flush with a tip end surface of the flexible tube or slightly protrudes from the tip end surface of the flexible tube, wherein the flexible tube includes a tip end curved portion, the hollow grip includes: a hollow grip main body; an indicator protrusion portion related to a curved direction of the tip end curved portion of the flexible tube and extending in a direction of a tip end of the flexible tube at an axially central portion of the hollow grip main body; and an anti-slip cylindrical cover portion covering the axially central portion of the hollow grip main body including the indicator protrusion portion, the cylindrical cover portion has high static friction property, transparency, and flexibility, and the indicator protrusion portion, which is related to the curved direction of the flexible tube and is covered by the cylindrical cover portion, is visually and tactilely recognizable from an outside of the cylindrical cover portion.

The embryo transfer instrument has favorable operability because, due to the axially central portion of the hollow grip main body including the indicator protrusion portion being covered by the anti-slip cylindrical cover portion, the fingers do not slip on the indicator protrusion portion when holding the hollow grip. In addition, the cylindrical cover portion has high static friction property, transparency, and flexibility, and the indicator protrusion portion that is related to the curved direction of the flexible tube and is covered by the cylindrical cover portion can be visually and tactilely recognized from the outside of the cylindrical cover portion, and thus the curved direction of the flexible tube can be easily checked.

Also, the embodiment described above may be configured as follows
(12) The embryo transfer instrument according to the above (11), further comprising: an embryo transfer tube member that is insertable into the flexible tube from which the stylet member has been removed, and includes a soft tip end portion that is protrudable from a tip end of the flexible tube by a predetermined length.
(13) The embryo transfer instrument according to the above (11) or (12), wherein the indicator protrusion portion protrudes in the curved direction of the tip end curved portion of the flexible tube.
(14) The embryo transfer instrument according to any one of the above (11) to (13), wherein the indicator protrusion portion covered by the cylindrical covering portion forms an indicator raised portion in the hollow grip.
(15) The embryo transfer instrument according to any one of the above (11) to (14), wherein the cylindrical covering portion is formed using an elastic rubber or an elastomer.
(16) The embryo transfer instrument according to any one of the above (11) to (15), wherein the cylindrical covering portion is formed using a transparent material or a translucent material, and the hollow grip main body is formed using a colored opaque material.
(17) The embryo transfer instrument according to any one of the above (11) to (16), wherein the cylindrical covering portion is fitted in an expanded state to the hollow grip main body, and adheres to an outer surface of the hollow grip main body by contraction.
(18) The embryo transfer instrument according to any one of the above (11) to (17), wherein the hollow grip main body includes a tip end-side annular bulging portion and a rear end-side annular bulging portion, and a tip end portion of the cylindrical covering portion covers a rear end portion of the tip end-side annular bulging portion and a rear end portion of the cylindrical covering portion covers a tip end portion of the rear end-side annular bulging portion so that two opposite ends of the cylindrical covering portion do not protrude from the hollow grip main body.
(19) The embryo transfer instrument according to any one of the above (11) to (18), wherein the hollow grip main body has a cross section that becomes smaller toward the axially center portion from a tip end portion and a rear end portion of the hollow grip main body, and the cylindrical covering portion adheres to an outer surface of the axially center portion of the hollow grip main body.
(20) The embryo transfer instrument according to any one of the above (11) to (19), wherein the stylet member includes: a shaft-shaped stylet main body; and a stylet hub that is fixed to a rear end of the stylet main body, and rotation restriction engaging portions that restrict relative rotation between the hollow grip and the stylet hub are provided at a rear end portion of the hollow grip and a tip end portion of the stylet hub, respectively.

## Claims

1. An oocyte retrieval needle for retrieving an oocyte from an ovarian follicle comprising:
a hollow needle having a blade tip with a puncture blade surface; and
a hollow grip is fixed to a rear end portion of the hollow needle,
wherein the hollow grip includes:
a hollow grip main body;
an indicator protrusion portion related to an orientation of the puncture blade surface and extending in a direction of the blade tip of the hollow needle at an axially central portion of the hollow grip main body; and
an anti-slip cylindrical cover portion covering the axially central portion of the hollow grip main body including the indicator protrusion portion,
the cylindrical cover portion has high static friction property, transparency and flexibility; and
the indicator protrusion portion, which is related to the orientation of the puncture blade surface and is covered by the cylindrical cover portion, is visually and tactilely recognizable from an outside of the cylindrical cover portion.

2. The oocyte retrieval needle according to claim 1,
wherein the puncture blade surface is offset from a position of the indicator protrusion portion of the hollow grip by approximately 90 degrees in a circumferential direction of the hollow needle so that, when the oocyte retrieval needle is held with the hollow grip being positioned on the right side, the blade tip being positioned on the left side, and the indicator protrusion portion facing upward, the puncture blade surface faces a front side.

3. The oocyte retrieval needle according to claim 1 or 2,
wherein the indicator protrusion portion covered by the cylindrical covering portion forms an indicator raised portion.

4. The oocyte retrieval needle according to any one of claims 1 to 3,
wherein the cylindrical covering portion is formed using an elastic rubber or an elastomer.

5. The oocyte retrieval needle according to any one of claims 1 to 4,
wherein the cylindrical covering portion is formed using a transparent material or a translucent material, and
the hollow grip main body is formed using a colored opaque material.

6. The oocyte retrieval needle according to any one of claims 1 to 5,
wherein the cylindrical covering portion is fitted in an expanded state to the hollow grip main body, and adheres to an outer surface of the hollow grip main body by contraction.

7. The oocyte retrieval needle according to any one of claims 1 to 6,
wherein the hollow grip main body includes a tip end-side annular bulging portion and a rear end-side annular bulging portion, and
a tip end portion of the cylindrical covering portion covers a rear end portion of the tip end-side annular bulging portion and a rear end portion of the cylindrical covering portion covers a tip end portion of the rear end-side annular bulging portion so that two opposite ends of the cylindrical covering portion do not protrude from the hollow grip main body.

8. The oocyte retrieval needle according to claim 7,
wherein the rear end portion of the tip end-side annular bulging portion that is covered by the tip end portion of the cylindrical covering portion has an increased diameter to form an increased diameter portion, and
the tip end portion of the rear end-side annular bulging portion that is covered by the rear end portion of the cylindrical covering portion has an increased diameter to form an increased diameter portion.

9. The oocyte retrieval needle according to claim 7 or 8,
wherein the tip end portion of the cylindrical covering portion has a thickness that decreases toward a tip end thereof, and
the rear end portion of the cylindrical covering portion has a thickness that decreases toward a rear end thereof.

10. The oocyte retrieval needle according to any one of claims 1 to 9,
wherein the hollow grip main body has a cross section that decreases toward the axially center portion from a tip end portion and a rear end portion of the hollow grip main body, and
the cylindrical covering portion adheres to an outer surface of the axially center portion of the hollow grip main body.

11. An embryo transfer instrument configured to be used to transfer an embryo into a living body comprising:
an outer tube member that includes a flexible tube that includes a passage that extends through from a tip end to a rear end thereof and a hollow grip that is fixed to a rear end portion of the flexible tube; and
a stylet member that is removably inserted into the outer tube member and whose tip end is substantially flush with a tip end surface of the flexible tube or slightly protrudes from the tip end surface of the flexible tube,
wherein the flexible tube includes a tip end curved portion,
the hollow grip includes:
a hollow grip main body;
an indicator protrusion portion that is related to a curved direction of the tip end curved portion of the flexible tube and extends in a direction of a tip end of the flexible tube at an axially center portion of the hollow grip main body; and
an anti-slip cylindrical covering portion that covers the axially center portion of the hollow grip main body including the indicator protrusion portion,
the cylindrical covering portion has high static friction property, transparency, and flexibility, and
the indicator protrusion portion that is related to the curved direction of the flexible tube and is covered by the cylindrical covering portion is visually and tactilely recognizable from an outside of the cylindrical covering portion.

12. The embryo transfer instrument according to claim 11, further comprising:
an embryo transfer tube member that is insertable into the flexible tube from which the stylet member has been removed, and includes a soft tip end portion that is protrudable from a tip end of the flexible tube by a predetermined length.

13. The embryo transfer instrument according to claim 11 or 12,
wherein the indicator protrusion portion protrudes in the curved direction of the tip end curved portion of the flexible tube.

14. The embryo transfer instrument according to any one of claims 11 to 13,
wherein the indicator protrusion portion covered by the cylindrical covering portion forms an indicator raised portion in the hollow grip.

15. The embryo transfer instrument according to any one of claims 11 to 14,
wherein the cylindrical covering portion is formed using an elastic rubber or an elastomer.

16. The embryo transfer instrument according to any one of claims 11 to 15,
wherein the cylindrical covering portion is formed using a transparent material or a translucent material, and
the hollow grip main body is formed using a colored opaque material.

17. The embryo transfer instrument according to any one of claims 11 to 16,
wherein the cylindrical covering portion is fitted in an expanded state to the hollow grip main body, and adheres to an outer surface of the hollow grip main body by contraction.

18. The embryo transfer instrument according to any one of claims 11 to 17,
wherein the hollow grip main body includes a tip end-side annular bulging portion and a rear end-side annular bulging portion, and
a tip end portion of the cylindrical covering portion covers a rear end portion of the tip end-side annular bulging portion and a rear end portion of the cylindrical covering portion covers a tip end portion of the rear end-side annular bulging portion so that two opposite ends of the cylindrical covering portion do not protrude from the hollow grip main body.

19. The embryo transfer instrument according to any one of claims 11 to 18,
wherein the hollow grip main body has a cross section that becomes smaller toward the axially center portion from a tip end portion and a rear end portion of the hollow grip main body, and
the cylindrical covering portion adheres to an outer surface of the axially center portion of the hollow grip main body.

20. The embryo transfer instrument according to any one of claims 11 to 19,
wherein the stylet member includes:
a shaft-shaped stylet main body; and
a stylet hub that is fixed to a rear end of the stylet main body, and
rotation restriction engaging portions that restrict relative rotation between the hollow grip and the stylet hub are provided at a rear end portion of the hollow grip and a tip end portion of the stylet hub, respectively.
